# EUROPEAN PATENT APPLICATION

(11) **EP 3 811 956 A1**
(43) Date of publication of application: **28.04.2021**
(21) Application number: 19827385.6
(22) Date of filing: 19.06.2019
(51) Int. Cl.: A61K 36/06, A23L 33/10, A61P 3/10, A61P 39/06, C12N 1/16, C12R 1/865

(54) **DIABETES-ALLEVIATING OR ANTIOXIDANT COMPOSITION COMPRISING YEAST EXTRACT AND METHOD FOR PREPARING YEAST EXTRACT**

(30) Priority: 25.06.2018 KR 20180072951
(71) Applicant: Coenbio Co., Ltd., Seongnam-si, Gyeonggi-do 13207 (KR)
(72) Inventor: YUM, Kyu Jin, Anyang-Si Gyeonggi-do 14106 (KR); HUR, Jae Seoun, Suncheon-Si Jeollanam-do 57973 (KR); PARK, Chan Ho, Yeosu-Si Jeollanam-do 59672 (KR)
(74) Representative: Cabinet Beau de Loménie
(86) International application number: PCT/KR2019/007381
(87) International publication number: WO 2020/004858

(57) **Abstract**

The present invention relates to a composition for ameliorating diabetes or enhancing antioxidant activity including a yeast extract and a method for preparing the yeast extract.

## Description

### [Technical Field]

The present invention relates to a composition for ameliorating diabetes or enhancing antioxidant activity including a yeast extract, a method for preparing the yeast extract, and a method for ameliorating diabetes or enhancing antioxidant activity.

### [Background Art]

With the recent increase in the prevalence of chronic diseases such as cancer, diabetes, hypertension, cardiac diseases, and cardiovascular diseases, studies using beneficial strains and their bioactive components have been conducted for the purposes of preventing and treating such chronic diseases. Reactive oxygen species and free radicals causing oxidative stress in modern people are persistently generated during *in vivo* metabolic processes in humans and readily react with other biomaterials due to their instability and high reactivity. Reactive oxygen species and free radicals attack biopolymers, degrading cell membranes and suppressing protein synthesis, thus causing mutation, cytotoxicity, and cancer.

### Natural bioactive co

mponents entering the body are known to have the function of preventing diabetes, cancer, etc. through inhibitory activity against oxidation. Thus, there is a need to develop natural antioxidants with high antioxidant activity that can relieve oxidative stress in modern people without causing side effects.

The incidence of diabetes as a chronic disease was less than 1% in the South Korean population in the 1970s. Since 2001, approximately one tenth of the South Korean population age 30 and up suffers from diabetes. Diabetes is the fifth leading cause of death in South Korea. Particularly, since diabetes causes complications such as retinopathy and cataract, therapeutic approaches for diabetes should be able to retard the digestion and uptake of carbohydrates such that complications are avoided while lowering blood glucose levels after a meal. Hypoglycemic agents such as α-glucosidase inhibitors (e.g., acarbose and voglibose) pose the risk of resistance and side effects such as weight gain, edema, nausea, and digestive disorder. Thus, there is a need to develop glucose-lowering agents based on natural products.

### [Disclosure]

### [Technical Problem]

It is an aspect of the present invention to provide a yeast extract that has the function of lowering or controlling blood glucose levels, or has an antioxidant activity
without causing side effects, and a composition including the yeast extract.

It is another aspect of the present invention to provide a method for preparing a yeast extract with antioxidant or antidiabetic activity.

### [Technical Solution]

One aspect of the present invention relates to a method for preparing a yeast extract for ameliorating diabetes or enhancing antioxidant activity, including: culturing a yeast and separating the culture into biomass and a culture fluid by centrifugation; dissolving the biomass to produce a strain lysate and extracting the strain lysate and/or the culture fluid with an organic solvent to produce an extract from the strain lysate and/or an extract from the culture fluid, or dissolving the biomass in an organic solvent to produce an extract from the strain lysate and/or adding an organic solvent to the culture fluid to produce an extract from the culture fluid; and concentrating and drying the extract.

Yeast is a microorganism used to make various alcoholic beverages, for example, beer, wine, and makgeolli, a traditional Korean rice wine. Yeast is a collective name for unicellular organisms that belong to groups of fungi and mushrooms but have no mycelia, do not have the ability to photosynthesize, and are not motile. A microorganism belonging to the genus *Saccharomyces* can be used as the yeast in the present invention. Specifically, the yeast is *Saccharomyces servazzii* or *Saccharomyces cerevisiae* but is not limited thereto. For example, the yeast may be a *Saccharomyces servazzii* strain (KCCM12157P), which was isolated from kimchi, a traditional Korean fermented vegetable food, and is effective in lowering blood glucose levels or enhancing antioxidant activity.

A natural or synthetic medium can be used to culture the yeast. Examples of carbon sources of the medium include, but are not limited to, glucose, sucrose, dextrin, dextrose, glycerol, and starch. Examples of nitrogen sources of the medium include, but are not limited to, peptone, meat extract, whole milk powder, yeast extract, dried yeast, soybean, ammonium salt, nitrate, and other organic and inorganic nitrogenous compounds. One or more inorganic salts may be added to the medium. Examples of such inorganic salts include, but are not limited to, magnesium, manganese, calcium, iron, and potassium salts. The medium may further include one or more compounds selected from amino acids, vitamins, nucleic acids, and compounds related thereto. The yeast strain may be cultured at a temperature of 20°C to 40°C, specifically 21°C to 30°C for 12 hours to 7 days, for example, 12 hours to 3 days. As an example, one or more galenic preparations, an extract thereof or a mixture thereof may be added to and cultured in the culture medium. Examples of the galenic preparations include *Acanthopanax senticosus* root bark, guava, baical skullcap root, amur cork-tree bark, cape jasmine fruit, schisandra fruit, coptis rhizome, and Solomon's seal rhizome. The use of the culture further improves the effect of the strain to lower or control blood glucose levels or enhance antioxidant activity. The addition of *Acanthopanax senticosus* root bark or baical skullcap root to the culture medium is particularly preferred. The *Acanthopanax senticosus* root bark or baical skullcap root may be added in an amount of 0.01% by weight (wt%) to 10 wt%, specifically 0.01 wt% to 5 wt%.

Thereafter, the strain cultured in the culture medium can be separated into biomass and a culture fluid by centrifugation. The culture fluid refers to a culture supernatant that is free of the biomass after centrifugation. The biomass contains the strain and can be obtained by removal of the culture supernatant or subsequent concentration of the residue. The composition of the culture fluid or the biomass may further include not only one or more typical ingredients necessary for the culture of the yeast but also one or more ingredients exerting a synergistic effect on the growth of the yeast. The composition of the culture fluid or the biomass can be readily determined by those skilled in the art.

An organic solvent capable of dissolving the biomass is added to the biomass to produce a strain lysate. The dissolution may be performed by a suitable process, for example, a mechanical process, to extract ingredients from the biomass. The mechanical process may be a process for homogenizing the biomass using a rotating blade at a high pressure, a process for disintegrating the biomass in a stirred mill, a process for pressing the sample through a narrow hole at a high pressure or a process using an ultrasonic homogenizer. Specifically, an ultrasonic homogenizer can be used to dissolve the biomass.

Alternatively, the biomass is dissolved by a suitable process, for example, a mechanical process, to produce a strain lysate, which is then extracted with an organic solvent to prepare an extract.

The organic solvent may be, for example, ethanol, butanol, chloroform, acetone, hexane or ethyl acetate. The use of ethanol, hexane or ethyl acetate, particularly hexane or ethyl acetate, as the extraction solvent is preferred in terms of blood glucose reduction. The use of hexane, ethyl acetate or ethanol as the extraction solvent is preferred in terms of antioxidant activity. The organic solvent may be used at a concentration of 30 wt% to 80 wt%, specifically 40 wt% to 70 wt%. The strain lysate and the culture fluid may be separately extracted with organic solvents or a mixture of the strain lysate and the culture fluid may be extracted with an organic solvent.

The extraction time and temperature may vary depending on design conditions. Specifically, the extraction can be performed at a temperature ranging from 20°C to 50°C for 1 hour to 12 hours. More specifically, the extraction can be performed at a temperature ranging from 20°C to 40°C or 25°C to 30°C for 1 hour to 6 hours. This temperature range is preferable in that active ingredients in the strain lysate or the culture fluid can be protected from thermal denaturation.

The extract may be optionally filtered. Examples of suitable filtration techniques include precipitation, membrane separation, centrifugation, fractional distillation, and distribution. For example, the extract may be filtered through a filter paper.

Next, the extract from the strain lysate or the culture fluid is concentrated and dried to obtain a yeast extract. For example, evaporation concentration, freezing concentration, membrane concentration or drying concentration may be used to concentrate the extract from the strain lysate or the culture fluid. Specifically, the extract from the strain lysate or the culture fluid can be concentrated under reduced pressure using a rotary evaporator. The concentrate can be dried by any suitable technique known in the art. Examples of suitable drying techniques include, but are not limited to, air drying, natural drying, spray drying, and freeze drying. Specifically, the concentrate can be freeze-dried.

Another aspect of the present invention relates to a composition for ameliorating diabetes, lowering blood glucose levels or enhancing antioxidant activity including a yeast extract prepared by the method described herein.

The yeast extract means an extract obtained by extracting the strain lysate and/or the culture fluid descried herein with an organic solvent. The yeast species, the organic solvent type, and the extraction process are the same as those described in the previous embodiment. The composition may be a food or pharmaceutical composition for lowering or controlling blood glucose levels, preventing diabetes or enhancing antioxidant activity. The composition of the present invention is useful in the treatment of cancer, inflammation, anemia, cardiac infarction, arteriosclerosis, diabetes, cerebral palsy, arthritis (*e.g*., rheumatoid arthritis), Parkinson's disease or autoimmune disease due to antioxidant activity thereof. One embodiment of the food composition may be a health food, health supplement food or health functional food composition. One embodiment of the pharmaceutical composition may be a quasidrug or a pharmaceutical preparation.

As an example, the composition may further include one or more antidiabetically or antioxidatively active ingredients selected from galenic preparations such as *Acanthopanax senticosus* root bark, guava, baical skullcap root, amur cork-tree bark, cape jasmine fruit, schisandra fruit, coptis rhizome, and Solomon's seal rhizome, extracts thereof, and mixtures thereof.

The composition of the present invention may include 1 wt% to 70 wt%, specifically 5 wt% to 60 wt% of the yeast extract, based on the total solid content thereof.

The yeast extract may be in a liquid or dry state. Specifically, the yeast extract is in the form of a dry powder.

For example, the composition may further include a pharmaceutically or sitologically acceptable carrier. In this case, the composition may be formulated with the carrier to provide a food or pharmaceutical drug.

As used herein, the term "pharmaceutically or sitologically acceptable carrier" refers to a carrier or diluent that causes no irritation to target organisms and does not deteriorate the biological activity and properties of the active ingredient.

The composition may be formulated into various preparations for oral or parenteral administration. Preferably, the composition is formulated into a liquid oral solution.

When formulated into a liquid solution, the pharmaceutically or sitologically acceptable carrier may be selected from saline solution, sterilized water, buffered saline, albumin injection solution, dextrose solution, maltodextrin solution, glycerol, and mixtures thereof that are sterile and biocompatible. If necessary, one or more general additives such as antioxidants, buffer solutions, and bacteriostatic agents may be added to the composition. The composition of the present invention may be formulated into liquid preparations (such as aqueous solutions, suspensions, and emulsions), pills, capsules, granules, and tablets. In this case, the composition of the present invention may further include one or more additives selected from diluents, dispersants, surfactants, binders, and lubricants. A binding agent, an emulsifier or preservative may be further added to the composition to prevent the quality of the composition from deteriorating. The composition of the present invention may further include one or more additives selected from amino acids, vitamins, enzymes, flavoring agents, non-protein nitrogen compounds, silicates, buffers, extractants, and oligosaccharides.

Examples of preparations including the composition of the present invention include tablets, troches, lozenges, water-soluble or oily suspensions, powders, granules, emulsions, hard or soft capsules, syrups, and elixirs. The composition of the present invention may be formulated into desired preparations such as tablets and capsules. In this case, the composition may include: a binder such as lactose, saccharose, sorbitol, mannitol, starch, amylopectin, cellulose or gelatin; an excipient such as dicalcium phosphate; a disintegrant such as corn starch or sweet potato starch; or a lubricant such as magnesium stearate, calcium stearate, sodium stearyl fumarate or polyethylene glycol wax. For capsule preparations, the composition may further include a liquid carrier such as a fatty oil.

The daily dose of the composition or the yeast extract may vary depending on the weight, age, sex, and health condition of the subject, main symptoms to be treated, prevented or ameliorated, time and mode of administration, and severity of the disease and may be in the range of about 0.0001 mg/kg to about 10 g/kg, specifically about 0.01 mg/kg to about 500 mg/kg. The composition or the yeast extract may be administered or applied 1 to 6 times, for example, 1 to 4 times, a day.

A further aspect of the present invention relates to a method for ameliorating, preventing or treating diabetes or enhancing antioxidant activity, which includes administering to a subject a therapeutically or prophylactically effective amount of a yeast extract prepared by the method described herein. The therapeutically or prophylactically effective amount may vary depending on the weight, age, sex, and health condition of the subject, main symptoms to be treated, prevented or ameliorated, time and mode of administration, and severity of the disease and may be in the range of about 0.0001 mg/kg to about 10 g/kg, specifically about 0.01 mg/kg to about 500 mg/kg, as described above. The therapeutically or prophylactically effective amount may be administered or applied 1 to 6 times, for example, 1 to 4 times, a day.

Embodiments of the present invention provide a composition for preventing or treating diabetes or enhancing antioxidant activity including a *Saccharomyces servazzii* strain (Ceb-kc-011), a culture thereof, a strain lysate thereof, a concentrate thereof, a dried product thereof or an extract thereof. The strain was deposited under the deposit number KCCM12157P. For example, the composition for enhancing antioxidant activity may exert therapeutic activity against cancer, aging, inflammation, anemia, cardiac infarction, arteriosclerosis, diabetes, cerebral palsy, arthritis (e.g., rheumatoid arthritis), Parkinson's disease or autoimmune disease.

### [Advantageous Effects]

A yeast extract prepared by the method according to the present invention has an outstanding ability to inhibit α-glucosidase, causes few side effects, and is safe. Due to these advantages, the yeast extract is highly valuable for the prevention and/or treatment of diabetes.

In addition, due to its superior antioxidant activity, the yeast extract is suitable for use in the prevention or treatment of diseases that are caused by excessive accumulation of free radicals and reactive oxygen species.

### [Description of Drawings]

FIG. 1 compares the α-glucosidase inhibition activities of extracts obtained by extraction of a strain lysate and a culture fluid with different solvents and acarbose as an α-glucosidase inhibitor in Example 3.
FIG. 2 shows the effects of extracts obtained by extraction of a strain lysate and a culture fluid with different solvents on the blood glucose levels of (2a) normal SD-rats and (2b) diabetes-induced SD-rats in Example 4 (CFP: strain lysate (phosphate buffer solution), CFE: strain lysate (ethanol), CFEA: strain lysate (ethyl acetate), SPM: culture fluid, SPE: culture fluid (ethanol), SPEA: culture fluid (ethyl acetate)).
FIG.3 compares the results of free radical scavenging assay for extracts obtained by extraction of a strain lysate and a culture fluid with different solvents and ascorbic acid as an antioxidant in Example 5, demonstrating antioxidant activities of the extracts.
FIG. 4 compares the results of radical cation de-colorization assay for extracts obtained by extraction of a strain lysate and a culture fluid with different solvents and ascorbic acid as an antioxidant in Example 5, demonstrating antioxidant activities of the extracts.
FIG. 5 compares the results of reducing power assay for extracts obtained by extraction of a strain lysate and a culture fluid with different solvents and ascorbic acid as an antioxidant in Example 5, demonstrating antioxidant activities of the extracts.
FIG. 6 compares the α-glucosidase inhibition activities of extracts obtained by extraction of a strain lysate and a culture fluid with different solvents and acarbose as an α-glucosidase inhibitor in Example 3.
FIG. 7 compares the results of free radical scavenging assay for extracts obtained by extraction of a strain lysate and a culture fluid with different solvents and ascorbic acid as an antioxidant in Example 5, demonstrating antioxidant activities of the extracts.
FIG. 8 compares the results of radical cation de-colorization assay for extracts obtained by extraction of a strain lysate and a culture fluid with different solvents and ascorbic acid as an antioxidant in Example 5, demonstrating antioxidant activities of the extracts.
FIG. 9 compares the results of reducing power assay for extracts obtained by extraction of a strain lysate and a culture fluid with different solvents and ascorbic acid as an antioxidant in Example 5, demonstrating antioxidant activities of the extracts.

### [Mode for Invention]

The present invention will be explained in more detail with reference to the following examples. However, these examples are provided for illustrative purposes only and the scope of the present invention is not limited thereto.

### Example 1: Preparation of yeast extracts 1

### (1) Sampling and strain isolation

A sample was taken from kimchi, a traditional Korean fermented food, diluted stepwise, plated on yeast extract peptone dextrose (YPD) supplemented with 1% sodium chloride, and cultured at 37°C for 24 h. A dominant strain was isolated from the sample. Colonies were selected and passaged three times in fresh media. The pure cultured strain was placed in a medium supplemented with 20% glycerol and stored at ≤ -70°C.

### (2) Investigation of taxological properties

The isolated strain was identified. To this end, the taxological properties of the strain were analyzed by 18s rRNA partial sequencing. As a result, the strain was found to have the sequence set forth in SEQ ID NO: 1 and have a homology of 99% with *Saccharomyces servazzii.*

The newly isolated strain *Saccharomyces servazzii* Ceb-kc-011 was deposited with the Korean Culture Center of Microorganisms (120-861, Hongje-2ga-gil, Seodaemun-Gu, Seoul, Korea) on November 10, 2017 and assigned accession number KCCM12157P.

The strain was freeze-dried and powdered.

### (3) Preparation of yeast extracts 1

*Saccharomyces servazzii* Ceb-kc-011 was cultured by the following procedure.

2 kg of dextrose, 1.5 kg of whole milk powder, 0.1 kg of peptone, and 0.1 kg of a commercial yeast extract were added to 100 liters of purified water. The mixture was sterilized in an autoclave at 121°C for 15-30 min and cooled to around 35°C. Thereafter, the sterile mixture was inoculated with 0.2-0.4 liters of *Saccharomyces servazzii* Ceb-kc-011, followed by culture with supply of air at around 35°C for 2-3 days.

Thereafter, the culture was separated into biomass and a culture fluid by centrifugation. The biomass and the culture fluid were extracted with the same amounts of different organic solvents (including ethanol, hexane, and ethyl acetate) by ultrasonic disintegration while maintaining a temperature of 25-30°C for 2 h. Each of the organic solvent extracts was produced in an amount of 0 mg/g-200 mg/g (dry weight) depending on the extraction solvent.

The organic solvent extracts from the strain and the organic solvent extracts from the culture fluid were concentrated under reduced pressure at 45°C and freeze-dried to prepare strain extracts (cell free) and culture fluid extracts (supernatant) as yeast extracts **1.**

### Example 2: Preparation of yeast extracts 2

Strain extracts and culture fluid extracts as yeast extracts **2** were prepared in the same manner as in (3) of Example 1, except that *Saccharomyces cerevisiae* was used as a yeast strain.

### Example 3: Evaluation of α-glucosidase inhibition activities

The α-glucosidase inhibition activities of yeast extracts **1** (Example 1) and **2** (Example 2) were evaluated by the following procedure.

The yeast extracts **1** and **2** were diluted to concentrations of 20, 40, 80, and 100 mg/ml. 20 µl of each of the diluted yeast extracts, 40 µl of a 0.1 M phosphate buffer (pH 7.0), 20 µl of a substrate (5 mM p-nitrophenyl-α-D-glucopyranoside, pH 7.0), and 20 µl of 1.0 unit/ml of α-glucosidase (Sigma) were homogenized. The mixture was allowed to react in an incubator at 37°C for 20 min. The reaction was quenched with 50 µl of Na₂CO₃ and the absorbance was measured at 405 nm. Inhibition (%) was calculated by (A-B/A) × 100 (%), where A is the absorbance in the absence of the inhibitor and B is the absorbance in the presence of the inhibitor.

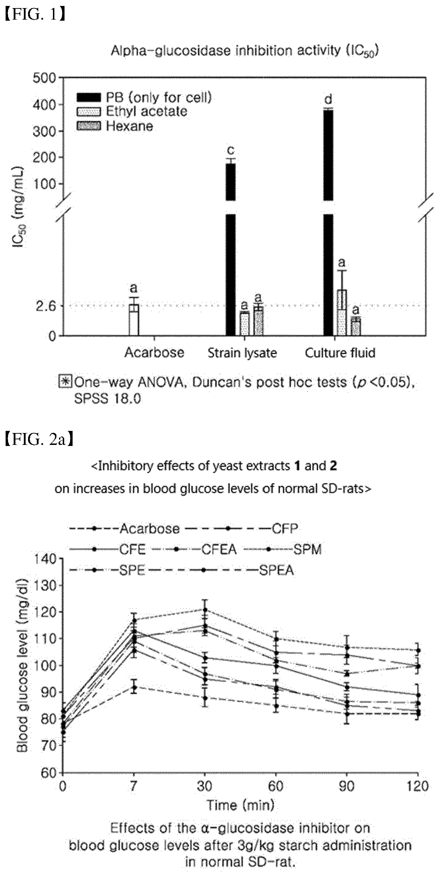

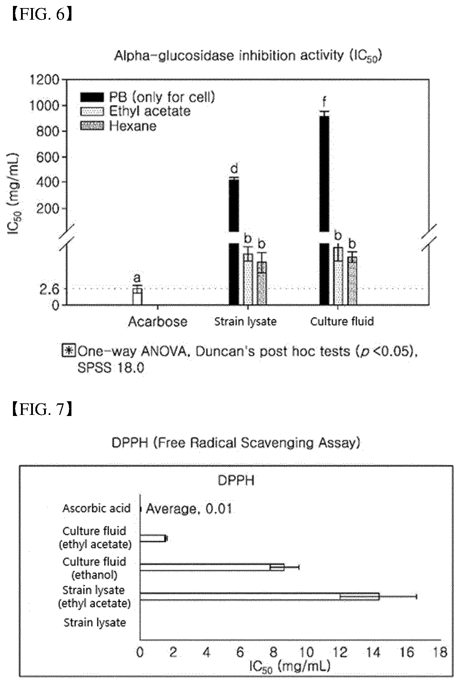

The α-glucosidase inhibitions of the extracts at different concentrations were calculated from regression curves plotted by entering data into the statistical program (SPSS 17.0) and were expressed as IC₅₀ values. IC₅₀ refers to the concentration of the extract required for 50% inhibition of enzyme activity. The results are shown in Tables 1 and 2 (see FIGs. 1 and 6).

**[Table 1] α-glucosidase inhibition activities of yeast extracts 1 (IC₅₀, mg/mL)**

| | PB (only for cell) | Ethyl acetate | Hexane |
|---|---|---|---|
| Acarbose | 2.6±0.6^{a} | - | - |
| Strain lysate | 174.2±21.3^{c} | 1.9±0.1^{a} | 2.4±0.3^{a} |
| Culture fluid | 376.6±6.9^{d} | 3.8±1.6^{a} | 1.4±0.2^{a} |

**[Table 2] α-glucosidase inhibition activities of yeast extracts 2 (IC₅₀, mg/mL)**

| | PB (only for cell) | Ethyl acetate | Hexane |
|---|---|---|---|
| Acarbose | 2.6±0.6 | - | - |
| Strain lysate | 421.2±15.2 | 8.4±1.2 | 6.9±1.7 |
| Culture fluid | 915.1±41.9 | 9.5±2.3 | 7.8±0.9 |

In Tables 1 and 2, PB represents treatment with phosphate buffer solution instead of the organic solvents.

The results in Tables 1 and 2 revealed that the strain lysate and the culture fluid *per se* were not active in inhibiting α-glucosidase but the ethyl acetate and hexane extracts were effective in inhibiting α-glucosidase.

### Example 4: Evaluation of abilities to lower blood glucose levels: Rat sugar loading test

6 week old male SD-rats (180-200 g) as experimental animals were acclimated to the vivarium for 5 days with *ad libitum* access to food (20 g) and water while maintaining the temperature at 25°C and the relative humidity (RH) at 60% under a 12 h light/dark cycle. The experimental animals were randomly divided into normal and diabetes-induced groups. The diabetes-induced groups were again divided into control and experimental groups. A solution of streptozotocin (STZ, 60 mg/kg, Sigma) as a diabetes inducing drug in a 0.1 M citrate buffer (pH 4.5) was once injected intraperitoneally into each fasted SD-rat. 3 days after injection, the blood glucose levels were measured using a glucometer. Only SD-rats whose blood glucose levels were more than 300 mg/dl were selected and used for experiments.

An experiment was conducted to investigate the abilities of yeast extracts **1** (Example 1) and **2** (Example 2) to inhibit increases in the blood glucose levels of the SD-rats. Each yeast extract (100 mg/kg), acarbose (20 mg/kg), and starch (3 g/kg) were orally administered and time-dependent changes in the blood glucose levels were measured.

The results are shown in FIGs. 2a (normal groups) and 2b (diabetes-induced groups).

The results in FIG.2a revealed that SPEA (the ethyl acetate extract from the culture fluid), CFEA (the ethyl acetate extract from the strain lysate), CFE (the ethanol extract from the strain lysate), and SPE (the ethanol extract from the culture fluid) were effective in inhibiting increases in the blood glucose levels caused by digestion and uptake of the starch in the normal groups (normal rats) whereas SPM (the culture fluid) and CFP (the strain lysate in the phosphate buffer solution) *per se* were not significantly effective in inhibiting increases in blood glucose levels.

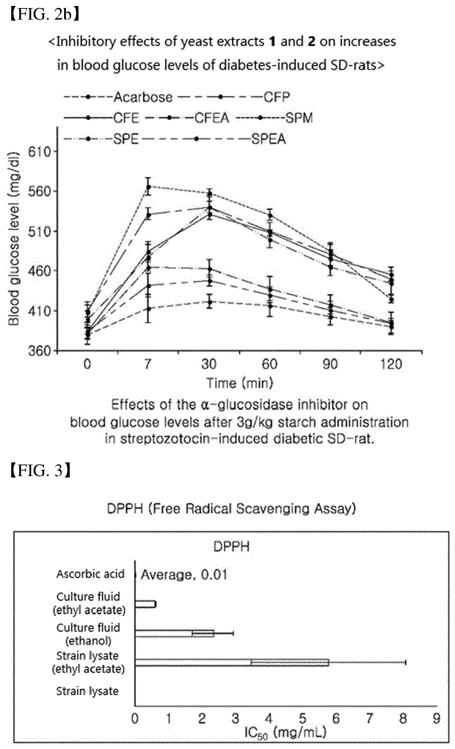

The results in FIG.2b revealed that the changes in the blood glucose levels of the diabetes-induced SD-rats were drastic compared to those of the normal groups, SPEA (the ethyl acetate extract from the culture fluid) and CFEA (the ethyl acetate extract from the strain lysate) caused the greatest reduction in blood glucose levels, and CFE (the ethanol extract from the strain lysate) and SPE (the ethanol extract from the culture fluid) inhibited marked initial increases in the blood glucose levels, whereas SPM (the culture fluid) and CFP (the strain lysate in the phosphate buffer solution) *per se* were not significantly effective in inhibiting increases in blood glucose levels.

### Example 5: Evaluation of antioxidant activities

The antioxidant activities of yeast extracts **1** (Example 1) and **2** (Example 2) were evaluated by the following procedures.

### (1) Free radical scavenging assay using DPPH

A solution of 2,2-diphenyl-1-picrylhydrazyl (DPPH) radicals in an organic solvent has a maximum absorbance at 515 nm. An antioxidant scavenges DPPH radicals, losing its original color and being made transparent.

The test sample was mixed with 2,2-diphenyl-1-picrylhydrazyl (DPPH, 100 µM) in a ratio of 1:20 and the mixture was stored at 37°C for 30 min. After completion of the reaction, the absorbance was measured at 517 nm using a spectrophotometer. A lower absorbance indicates a higher antioxidant activity.

The DPPH scavenging activities of yeast extracts **1** and **2** were expressed as IC₅₀ values (corresponding to the concentrations of the extracts required to scavenge 50% of the radicals). The results are shown in Tables 3 and 4 (see FIGs. 3 and 7).

**[Table 3] DPPH scavenging activities of yeast extracts 1**

| (IC₅₀, mg/mL) | AVERAGE | STDEV |
|---|---|---|
| Strain lysate | 0.00 | 0.00 |
| Strain lysate (ethyl acetate) | 5.78 | 2.30 |
| Culture fluid (ethanol) | 2.32 | 0.61 |
| Culture fluid (ethyl acetate) | 0.62 | 0.03 |
| Ascorbic acid | 0.01 | 0.00 |

**[Table 4] DPPH scavenging activities of yeast extracts 2**

| (IC₅₀, mg/mL) | AVERAGE | STDEV |
|---|---|---|
| Strain lysate (ethyl acetate) | 14.30 | 2.31 |
| Culture fluid (ethanol) | 8.65 | 0.85 |
| Culture fluid (ethyl acetate) | 1.55 | 0.07 |
| Ascorbic acid | 0.01 | 0.00 |

The results in Tables 3 and 4 revealed that each of the ethyl acetate extracts from the culture fluids showed the highest antioxidant activity comparable to that of ascorbic acid, and the ethanol extracts from the culture fluids and the ethyl acetate extracts from the strain lysates followed in this order. Each of the strain lysates *per se* showed no antioxidant activity.

### (2) Radical cation de-colorization assay using ABTS

ABTS scavenging activities were measured as follows.

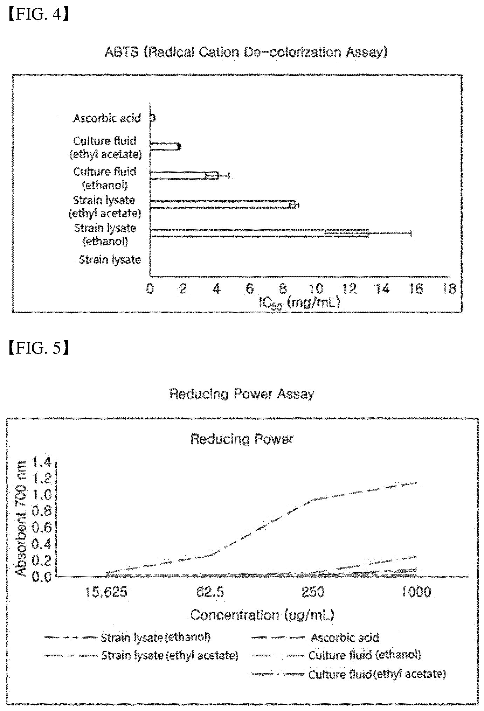

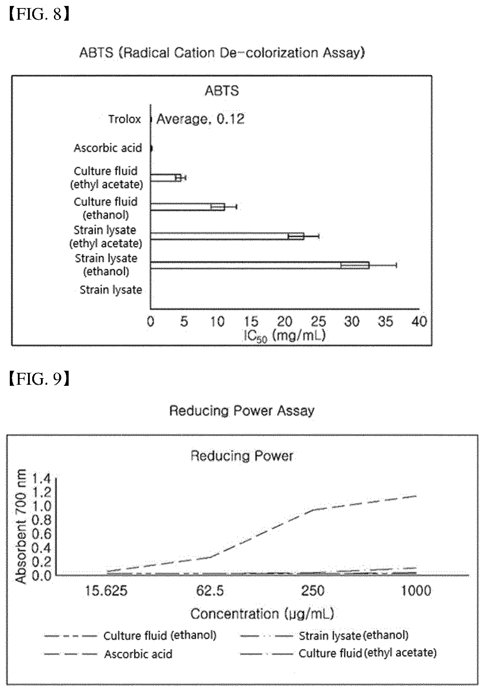

7 mM ABTS was mixed with 2.45 mM potassium persulfate and incubated at room temperature for 16 h to create ABTS cations (ABTS⁺). The mixture was diluted until an absorbance of ≤ 0.7 at 734 nm was reached. 100 µl of each sample was added to 100 µl of the ABTS⁺ solution. After 3 min, the absorbance was measured. The ABTS scavenging activities of yeast extracts **1** and **2** were expressed as IC₅₀ values (corresponding to the concentrations of the extracts required to scavenge 50% of the cations). The results are shown in Tables 5 and 6 (see FIGs. 4 and 8).

**Table 5] ABTS scavenging activities of yeast extracts 1**

| (IC₅₀, mg/mL) | AVERAGE | STDEV |
|---|---|---|
| Strain lysate | 0.00 | 0.00 |
| Strain lysate (ethanol) | 13.12 | 2.61 |
| Strain lysate (ethyl acetate) | 8.68 | 0.27 |
| Culture fluid (ethanol) | 4.05 | 0.70 |
| Culture fluid (ethyl acetate) | 1.73 | 0.05 |
| Ascorbic acid | 0.26 | 0.03 |

**[Table 6] ABTS scavenging activities of yeast extracts 2**

| (IC₅₀, mg/mL) | AVERAGE | STDEV |
|---|---|---|
| Strain lysate | 0.00 | 0.00 |
| Strain lysate (ethanol) | 32.50 | 4.10 |
| Strain lysate (ethyl acetate) | 22.80 | 2.30 |
| Culture fluid (ethanol) | 11.00 | 1.90 |
| Culture fluid (ethyl acetate) | 45.0 | 0.70 |
| Ascorbic acid | 0.26 | 0.03 |

The results in Table 5 revealed that the ethyl acetate extract from the culture fluid showed the highest antioxidant activity against ABTS comparable to that of ascorbic acid, and the ethanol extract from the culture fluid, the ethyl acetate extract from the strain lysate, and the ethanol extract from the strain lysate followed in this order. The results in Table 6 revealed that the ethyl acetate extract from the culture fluid showed the highest antioxidant activity against ABTS. Each of the strain lysates *per se* showed no antioxidant activity.

### (3) Reducing power assay

The antioxidant activities of yeast extracts **1** and **2** were evaluated by reducing power assay. This assay was performed by the following procedure.

First, 50 µL of potassium ferricyanide and 20 µL of each sample were added to 50 µL of a 0.2 M phosphate buffer solution (pH 6.6) and the mixture was allowed to react at 50°C for 20 min. After completion of the reaction, 50 µL of a 10% TCA solution was added, followed by centrifugation at 3000 × g for 20 min. 100 µL of the supernatant, 200 µL of triple-distilled water, and 20 µL of 0.1% ferric chloride were added, and the absorbance was measured at 700 nm. Trolox and BHA were used as positive controls.

The results are shown in Tables 7 and 8 (see FIGs. 5 and 9).

**[Table 7] Reducing power activities of yeast extracts 1**

| (µg/mL) | Strain lysate | Strain lysate (ethanol) | Strain lysate (ethyl acetate) | Culture fluid (ethanol) | Culture fluid (ethyl acetate) | Ascorbic acid |
|---|---|---|---|---|---|---|
| 15.625 | 0.002±0.001 | 0.001±0.001 | 0.002±0.001 | 0.003±0.002 | 0.004±0.001 | 0.058±0.001 |
| 62.5 | 0.001±0.001 | 0.002±0.001 | 0.004±0.000 | 0.003±0.001 | 0.015±0.001 | 0.267±0.002 |
| 250 | 0.001±0.001 | 0.005±0.001 | 0.019±0.002 | 0.016±0.001 | 0.060±0.001 | 0.940±0.005 |
| 1000 | 0.003±0.001 | 0.021±0.004 | 0.089±0.001 | 0.079±0.002 | 0.251±0.004 | 1.146±0.008 |

**[Table 8] Reducing power activities of yeast extract 2**

| (µg/mL) | Strain lysate | Strain lysate (ethanol) | Strain lysate (ethyl acetate) | Culture fluid (ethanol) | Culture fluid (ethyl acetate) | Ascorbic acid |
|---|---|---|---|---|---|---|
| 15.625 | 0.000±0.000 | 0.000±0.001 | 0.000±0.001 | 0.001±0.000 | 0.001±0.000 | 0.058±0.001 |
| 62.5 | 0.000±0.000 | 0.000±0.001 | 0.001±0.001 | 0.001±0.001 | 0.004±0.00 | 0.267±0.002 |
| 250 | 0.001±0.001 | 0.002±0.001 | 0.005±0.002 | 0.005±0.002 | 0.020±0.002 | 0.940±0.005 |
| 1000 | 0.002±0.001 | 0.005±0.002 | 0.022±0.003 | 0.021±0.003 | 0.104±0.004 | 1.146±0.008 |

## Claims

1. A method for preparing a yeast extract for ameliorating diabetes or enhancing antioxidant activity, comprising: culturing a yeast and separating the culture into biomass and a culture fluid by centrifugation; dissolving the biomass to produce a strain lysate and extracting the strain lysate and/or the culture fluid with an organic solvent to produce an extract from the strain lysate and/or an extract from the culture fluid, or dissolving the biomass in an organic solvent to produce an extract from the strain lysate and/or adding an organic solvent to the culture fluid to produce an extract from the culture fluid; and concentrating and drying the extract.

2. The method according to claim 1, wherein the organic solvent is butanol, chloroform, acetone, ethanol, ethyl acetate or hexane.

3. The method according to claim 1, wherein culturing is performed at a temperature of 20°C to 40°C for 12 hours to 7 days.

4. The method according to claim 1, wherein the yeast is *Saccharomyces servazzii* or *Saccharomyces cerevisiae.*

5. The method according to claim 4, wherein the *Saccharomyces servazzii* is deposited under the deposit number KCCM12157P.

6. A composition for ameliorating diabetes or enhancing antioxidant activity comprising a yeast extract prepared by the method according to claim 1.

7. The composition according to claim 6, wherein the composition comprises 1 wt% to 70 wt% of the yeast extract, based on the total solid content thereof.

8. The composition according to claim 6, wherein the composition is a pharmaceutical or food composition.

9. A composition for ameliorating diabetes or enhancing antioxidant activity comprising an extract from a strain lysate or culture fluid of a yeast.

10. The composition according to claim 9, wherein the organic solvent is butanol, chloroform, acetone, ethanol, ethyl acetate or hexane.

11. The composition according to claim 9, wherein the yeast is *Saccharomyces servazzii* or *Saccharomyces cerevisiae.*

12. A *Saccharomyces servazzii* strain (Ceb-kc-011), a culture fluid thereof or a strain lysate thereof,
wherein the strain is deposited under the deposit number KCCM12157P.


<110>    COENBIO CO., Ltd.

<120>    A composition for improving diabetes or for use of antioxidant
         comprising yeast extract and a method for preparing the yeast
         extract

<130>    P19-6031

<150>    KR 18/072951
<151>    2018-06-25

<160>    1

<170>    KoPatentIn 3.0

<210>    1
<211>    1686
<212>    DNA
<213>    Artificial Sequence

<220>
<223>    A necleic acid sequence of Saccharomyces servazzii Ceb-kc-011


<400>    1
ttatacagtg aaactgcgaa tggctcatta aatcagttat cgtttatttg atagttcctt        60

tactacatgg tataactgtg gtaattctag agctaataca tgcttaaaat cccgaccttt       120

tggaagggat gtatttatta gataaaaaat caatgtcttc ggactctttg atgattcata       180

ataacttttc gaatcgcatg gccttgtgcc ggcgatggtt cattcaaatt tctgccctat       240

caactttcga tggtaggata gtggcctacc atggtttcaa cgggtaacgg ggaataaggg       300

ttcgattccg gagagggagc ctgagaaacg gctaccacat ccaaggaagg cagcaggcgc       360

gcaaattacc caatcctaat acagggaggt agtgacaata aataacgata cagggccctt       420

tcgggtcttg taattggaat gagtacaatg taaatacctt aacgaggaac aattggaggg       480

caagtctggt gccagcagcc gcggtaattc cagctccaat agcgtatatt aaagttgttg       540

cagttaaaaa gctcgtagtt gaactttggg cctggttggc cggtctggct ttttgccacg       600

tactggaatg caaccgggcc tttccttctg gctaacctta ggctccttgt gggtctttgg       660

cgaaccagga cttttacttt gaaaaaatta gagtggttca aagcaggcgt attgctcgaa       720

tatattagca tggaataatg gaataggacg tttggttcta ttttgttggt ttctaggacc       780

atcgtaatga ttaataggga cggtcggggg catcagtatt caattgtcag aggtgaaatt       840

cttggattta ttgaagacta actactgcga aagcatttgc caaggacgtt ttcattaatc       900

aagaacgaaa gttaggggat cgaagatgat cagataccgt cgtagtctta accataaact       960

atgccgacta gggatcgggt ggtgtttttt taatgaccca ctcggcacct tacgagaaat      1020

caaagtcttt gggttctggg gggagtatgg tcgcaaggct gaaacttaaa ggaattgacg      1080

gaagggcacc accaggagtg gagcctgcgg cttaatttga ctcaacacgg ggaaactcac      1140

caggtccaga cacaataagg attgacagat tgagagctct ttcttgattt tgtgggtggt 1200

ggtgcatggc cgttcttagt tggtggagtg atttgtctgc ttaattgcga taacgaacga 1260

gaccttaacc tactaaatag tggtgctagc atttgctggt tcttccactt cttagaggga 1320

ctatcgattt caagtcgatg gaagtttgag gcaataacag gtctgtgatg cccttagacg 1380

ttctgggccg cacgcgcgct acactgacgg agccagcgag tctaacctag gccgagaggt 1440

cctggtaatc ttgtgaaact ccgtcgtgct ggggatagag ctttgtaatt tttgctcttc 1500

aacgaggaat tcctagtaag cgcaagtcat cagcttgcgt tgattacgtc cctgcccttt 1560

gtaccaccgc ccgtcgctag taccgattga atggcttagt gaggcctcag gatctgctta 1620

gagaaggggg caactccatc tcagagcgga aaatctggtc aaacttggtc atttagagaa 1680

ctaaac 1686